# EUROPÄISCHE PATENTSCHRIFT

(11) **EP 1 421 910 B1**
(45) Veröffentlichungstag und Bekanntmachung des Hinweises auf die Patenterteilung: **12.09.2007**
(21) Anmeldenummer: 03025648.1
(22) Anmeldetag: 07.11.2003
(51) Int. Cl.: A61B 17/28

(54) **Chirurgisches Instrument**
Surgical instrument
Instrument chirurgical

(30) Priorität: 20.11.2002 DE 10255083
(43) Veröffentlichungstag der Anmeldung: 26.05.2004
(73) Patentinhaber: Aesculap AG & Co. KG, 78532 Tuttlingen (DE)
(72) Erfinder: Dworschak, Manfred, 78589 Dürbheim (DE); Lutze, Theodor, 78582 Balgheim (DE); Morales, Pedro, 78532Tuttlingen-Nendingen (DE); Weisshaupt, Dieter, 78194 Immendingen (DE)
(74) Vertreter: Hoeger, Stellrecht & Partner Patentanwälte

(56) Entgegenhaltungen:
- DE-U- 20 112 920
- US-A- 3 175 556
- US-A- 5 571 120
- US-A- 5 938 667

## Beschreibung

Die Erfindung betrifft ein chirurgisches Instrument mit mindestens zwei relativ zueinander beweglichen Werkzeugelementen, mit mindestens einem den mindestens zwei Werkzeugelementen zugeordneten Betätigungselement und mit einer Sperrvorrichtung zum Festlegen einer relativen Stellung der mindestens zwei Werkzeugelemente, wobei die Sperrvorrichtung zwei aneinander abgleitende Sperrglieder umfaßt und wobei das eine Sperrglied einem der mindestens zwei Werkzeugelemente zugeordnet ist und das andere Sperrglied einem anderen der mindestens zwei Werkzeugelemente.

Die bei Instrumenten der eingangs beschriebenen Art vorgesehenen Sperrvorrichtungen dienen dazu, bei den Instrumenten, insbesondere mit Fassund/oder Greiffunktionen die Bewegung der beispielsweise als Maulteile ausgebildeten Werkzeugelemente für eine bestimmte Zeit zu unterbinden oder auszuschalten. Dies bedeutet, daß bei aktivierter Sperrvorrichtung die Werkzeugelemente diejenige Stellung beibehalten, welche sie im Moment der Aktivierung der Sperrvorrichtung innehatten.

Bekannt sind Sperrvorrichtungen, die ständig aktiv sind. Die Sperrelemente oder Sperrglieder gleiten in diesem Fall bei jeder vollen Betätigung des Instrumentes aneinander ab. Dies wird jedoch bei vielen Instrumenten als störend empfunden.

Ferner gibt es Sperrvorrichtungen, welche ein- und ausgeschaltet werden können. Dies bedeutet, daß bei einer sogenannten ausgeschalteten Sperrvorrichtung die Sperrglieder beim vollen Betätigen des Instrumentes nicht aneinander abgleiten. Als nachteilig hat sich dabei erwiesen, daß bei derartigen Instrumenten die Sperrvorrichtungen versehentlich aus- oder eingeschaltet werden können.

Es ist daher Aufgabe der vorliegenden Erfindung, ein gattungsgemäßes chirurgisches Instrument so zu verbessern, daß es einfacherer und sicherer handhabbar ist.

Diese Aufgabe wird bei einem chirurgischen Instrument der eingangs genannten Art erfindungsgemäß dadurch gelöst, daß das Instrument mindestens eine Entsperrungsvorrichtung zum irreversiblen Entsperren der mindestens zwei Sperrglieder voneinander umfaßt.

Mit einer solchen Entsperrungsvorrichtung ist es möglich, daß eine Sperrvorrichtung eines chirurgischen Instruments, welche ursprünglich ständig eingeschaltet war, wahlweise ausgeschaltet wird und dann endgültig in dem ausgeschalteten Zustand verbleibt. Bei manchen chirurgischen Eingriffen ist es gewünscht, daß die Sperrvorrichtung ständig eingeschaltet bleibt. In diesem Fall muß die Entsperrungsvorrichtung nicht betätigt werden. Ist es jedoch erwünscht, daß die Sperrvorrichtung dauernd ausgeschaltet sein soll, dann kann diese durch entsprechende Betätigung dauerhaft ausgeschaltet werden. Dadurch, daß chirurgische Instrumente aufgrund der Entsperrrungsvorrichtung grundsätzlich mit einer Sperrvorrichtung ausgestattet sein können, die wahlweise ausgeschaltet wird oder nicht, lassen sich insgesamt die Instrumentenvielfalt und die Herstellungskosten reduzieren.

Vorzugsweise ist die mindestens eine Entsperrungsvorrichtung plastisch verformbar. Diese Eigenschaft ermöglicht es, daß die Entsperrungsvorrichtung von einer ersten Stellung in eine zweite Stellung überführt werden kann, ohne in die erste Stellung selbständig zurückzukehren. Damit kann der gewählte, beispielsweise auch der geänderte Schaltzustand der Sperrvorrichtung auf einfache Weise beibehalten werden.

Günstig ist es, wenn die mindestens eine Entsperrungsvorrichtung eine Lösevorrichtung zum irreversiblen Lösen des mindestens einen Sperrglieds vom Instrument umfaßt. Beispielsweise kann das Sperrglied vom Instrument, z.B. mittels eines Betätigungsglieds abgebrochen werden. Damit wird verhindert, daß die Sperrvorrichtung unbeabsichtigt wieder eingeschalten wird.

Eine besonders vorteilhafte Ausgestaltung ergibt sich dann, wenn die Lösevorrichtung eine Sollbruchstelle umfaßt. Damit läßt sich das Sperrglied auf einfache Weise vom Instrument lösen, insbesondere abbrechen.

Grundsätzlich ist es denkbar, daß das Sperrglied auch bei einer in ihrem Schaltzustand veränderten Sperrvorrichtung am Instrument verbleibt. Dies kann vorteilhafterweise dadurch erreicht werden, daß die mindestens eine Entsperrungsvorrichtung eine Sollknickstelle umfaßt. Das Sperrglied wird in diesem Fall beispielsweise nur so weit relativ zum Instrument abgeknickt, daß es mit dem anderen Sperrglied nicht mehr in Wechselwirkung treten kann und die Sperrvorrichtung damit insgesamt funktionsunfähig wird.

Ein besonders einfacher Aufbau des Instruments ergibt sich, wenn das chirurgische Instrument zwei Betätigungsglieder umfaßt und wenn an jedem der beiden Betätigungsglieder ein Sperrglied angeordnet ist. Das Instrument kann in diesem Fall symmetrisch ausgebildet sein, wodurch die Vielfalt der zur Herstellung des Instruments erforderlichen Werkzeuge und damit auch die Herstellungskosten reduziert werden können.

Grundsätzlich kann es sich bei dem chirurgischen Instrument um ein beliebiges, mindestens zwei Werkzeugelemente und zugeordnete Betätigungselemente aufweisende Instrumente handeln. Beispiele hierfür sind Rohrschaftinstrumente mit einem starr am Rohrschaft angeordneten Betätigungsgriff und einem relativ zu diesem verschwenkbaren Betätigungsgriff zu Betätigung von an einem distalen Ende des Rohrschafts angeordneten Maulteilen unter Verwendung einer Schub- und Zugstange oder dergleichen. Vorzugsweise ist das mindestens eine Betätigungsglied einem mit dem mindestens einen Werkzeugelement verbundene Branche. Ein derartiges Instrument kann beispielsweise eine einfache Schere oder Zange sein, bei der zwei starre Instrumentenhälften im wesentlichen identisch ausgebildet sind.

Grundsätzlich könnte die Entsperrungsvorrichtung eines der beiden Werkzeugelemente mit einem der beiden Sperrglieder verbinden. Besonders vorteilhaft ist es jedoch, wenn die mindestens eine Entsperrungsvorrichtung eines der beiden Sperrglieder und eines der beiden Betätigungsglieder verbindet. Damit läßt sich eine Bewegung der beiden Werkzeugelemente indirekt über ein Sperren einer Relativbewegung eines der beiden Betätigungselemente sperren.

Bei einer bevorzugten Ausführungsform der Erfindung kann vorgesehen sein, daß die mindestens eine Entsperrungsvorrichtung aus Kunststoff hergestellt ist. Durch die Wahl dieses Werkstoffs läßt sich auf einfache Weise eine für die Aktivierung der Entsperrungsvorrichtung erforderliche Kraft einstellen. Zudem ist eine derartige Entsperrungsvorrichtung besonders günstig in der Herstellung.

Vorzugsweise ist das mindestens eine Sperrglied aus Kunststoff hergestellt. Ein derartiges Sperrglied läßt sich besonders einfach und kostengünstig herstellen, insbesondere auch in Verbindung mit einer aus Kunststoff hergestellten Entsperrungsvorrichtung an das Instrument anspritzen.

Eine einfache und vorteilhafte Verbindung der Betätigungselemente und der Sperrglieder läßt sich erreichen, wenn die mindestens eine Entsperrungsvorrichtung an eines der mindestens zwei Betätigungselemente und/oder an eines der beiden Sperrglieder angespritzt ist. Eine derart angeformte Entsperrungsvorrichtung läßt sich unabhängig von dem für den Grundkörper des Instruments verwendeten Werkstoff einfach und kostengünstig am Instrument anbringen.

Um die Entsperrungsvorrichtung besonders einfach und sicher betätigen zu können, ist es vorteilhaft, wenn die mindestens eine Entsperrungsvorrichtung eine Querschnittsfläche aufweist, die kleiner als die halbe Querschnittsfläche des mit ihr verbundenen Sperrglieds ist. Sie läßt sich in diesem Fall besonders leicht abknicken oder auch abbrechen.

Um ein Ausschalten der Sperrvorrichtung zusätzlich zu erleichtern, ist es günstig, wenn die mindestens eine Entsperrungsvorrichtung zwei voneinander beabstandete, eines der beiden Sperrglieder mit dem mindestens einen Betätigungselement verbindende Verbindungsstege umfaßt. Auf diese Weise lassen sich beispielsweise zwei getrennte Sollknickstellen oder Sollbruchstellen ausformen, die gemeinsam oder getrennt aktiviert werden können.

Damit eine Funktion der Sperrvorrichtung im eingeschalteten Zustand sichergestellt ist, kann bei einer bevorzugten Ausführungsform der Erfindung vorgesehen sein, daß jedes Sperrglied mindestens einen in Richtung auf das damit verbundene Betätigungsglied weisenden Rückhaltevorsprung aufweist und daß der mindestens eine Rückhaltevorsprung des einen Betätigungsglieds und der mindestens eine Rückhaltevorsprung des anderen Betätigungsglieds in einer Sperrstellung der Sperrvorrichtung aneinander anliegen. Damit lassen sich auf einfache Weise ineinander verhakende Vorsprünge an den Sperrgliedern ausbilden, die ein Öffnen beziehungsweise Schließen der Werkzeugelemente wirksam verhindern.

Damit mehrere unterschiedliche Stellungen der Sperrvorrichtung auswählbar sind, ist es vorteilhaft, wenn mindestens ein Sperrglied mehrere in Richtung auf die damit verbundene Branche weisende Rückhaltevorsprünge aufweist.

Vorzugsweise ist ein Grundkörper des Instruments aus Metall hergestellt und wahlweise mindestens teilweise von einem Kunststoff bedeckt. Der metallische Grundkörper erhöht die Stabilität des Instruments. Durch Verwendung von Kunststoff lassen sich das Gewicht des Instruments reduzieren und, aufgrund des kostengünstigeren Herstellungsmaterials, Herstellungskosten sparen.

Besonders einfach und kostengünstig wird die Herstellung des Instruments, wenn es vollständig aus Kunststoff hergestellt ist.

Die nachfolgende Beschreibung einer bevorzugten Ausführungsform der Erfindung dient im Zusammenhang mit der Zeichnung der näheren Erläuterung. Es zeigen:
- Figur 1:: eine Seitenansicht einer Tupferklemme mit einer daran angeordneten Sperre; und
- Figur 2:: eine Schnittansicht längs Linie 2-2 in Figur 1.

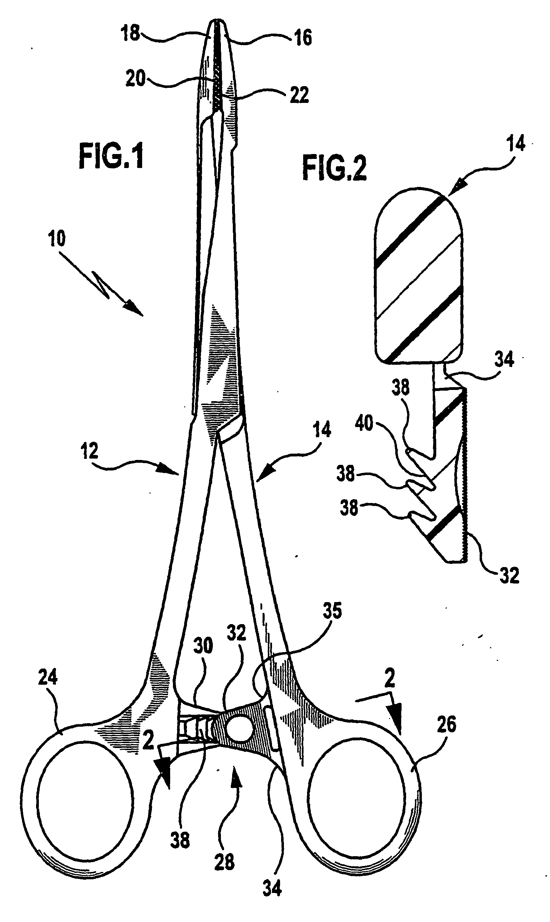

In Figur 1 ist eine insgesamt mit dem Bezugszeichen 10 versehene Tupferklemme dargestellt, die aus zwei identischen, aneinander verschwenkbar gelagerten Branchen 12 und 14 gebildet ist.

Jede der beiden Branchen 12 und 14 ist an ihrem distalen Ende mit einer Klemmbacke 16 beziehungsweise 18 versehen, die aufeinander zu weisende, aufgerauhte Klemmflächen 20 und 22 tragen. An Ihrem proximalen Ende sind die Branchen 12 und 14 jeweils mit einem Fingerring 24 beziehungsweise 26 versehen, durch die ein Operateur die Tupferklemme 10 greifen kann, beispielsweise indem er seinen Daumen durch den Fingerring 24 und seinen Zeigefinger durch den Fingerring 26 steckt.

Die Tupferklemme 10 ist mit einer insgesamt mit dem Bezugszeichen 28 versehenen Sperre versehen, die durch zwei identische, jeweils an einer der beiden Branchen 12 und 14 angeordnete Sperrglieder 30 und 32 gebildet wird.

Das Sperrglied 32 ist über zwei schmale, voneinander beabstandete Stege 34 und 35 mit der Branche 14 verbunden, das Sperrglied 30 dagegen geht direkt in die Branche 12 über. Jedes der beiden Sperrglieder 30 und 32 ist jeweils mit drei Sperrzähnen 38 versehen, die schräg vom jeweiligen Sperrglied 30 beziehungsweise 32 in Richtung auf die mit dem Sperrglied 30 beziehungsweise 32 verbundene Branche 12 beziehungsweise 14 hinweisen.

Die zwischen den Sperrzähnen 38 angeordneten Ausnehmungen 40 sind so ausgestaltet, daß jeweils ein Sperrzahn 38 des anderen Sperrglied in die Ausnehmung 40 eintauchen kann. Durch die Ausbildung von jeweils drei Sperrzähnen lassen sich insgesamt drei Stellung der Sperre 28 einstellen.

Durch die Verbindung des Sperrglieds 32 über Verbindungsstege 34 und 35 kann dieses auf einfache Weise aus der in Figur 1 dargestellten Stellung der Sperre 28 im eingeschalteten Zustand, in welchem die beiden Sperrglieder in Wechselwirkung treten können, abgeknickt oder sogar ganz abgebrochen werden, wodurch die Sperre 28 irreversibel ausgeschaltet wird. Die Stege 34 und 35 bilden eine einfache Entsperrungsvorrichtung zum Entsperren der Sperre 28. Das Sperrglied kann bei entsprechender Schwächung der Stege 34 und 35 abgebrochen werden. Die Stege 34 und 35 bilden dann jeweils Sollbruchstellen. Werden die Stege 34 und 35 jedoch etwas stabiler ausgestaltet, so läßt sich durch Abknicken derselben aufgrund einer plastischen Verformung der Stege 34 und 35 gleichfalls ein dauerhaftes Ausschalten der Sperre 28 erreichen.

Vorzugsweise ist die gesamte Tupferklemme aus Kunststoff hergestellt, so daß sich ein besonders einfacher Aufbau des Instruments und eine besonders einfache Herstellung ergibt. Es ist jedoch auch möglich, an eine aus Metall hergestellte Tupferklemme 10 Sperrglieder 30 und 32 in der oben beschriebenen Form aus Kunststoff anzuspritzen, und zwar direkt auf eine metallische Oberfläche oder auf bereits teilweise mit Kunststoff ummantelte Bereiche des Instruments.

## Patentansprüche

1. Chirurgisches Instrument (10) mit mindestens zwei relativ zueinander beweglichen Werkzeugelementen (16,18), mit mindestens einem den mindestens zwei Werkzeugelemente (16,18) zugeordneten Betätigungselement (12,14) und mit einer Sperrvorrichtung (28) zum Festlegen einer relativen Stellung der mindestens zwei Werkzeugelemente (16,18), wobei die Sperrvorrichtung (28) zwei aneinander abgleitende Sperrglieder (30,32) umfaßt und wobei das eine Sperrglied (30,32) einem der mindestens zwei Werkzeugelemente (16,18) zugeordnet ist und das andere Sperrglied (30,32) einem anderen der mindestens zwei Werkzeugelemente (16,18), **dadurch gekennzeichnet, daß** das Instrument (10) mindestens eine Entsperrungsvorrichtung (34, 35) zum irreversiblen Entsperren der mindestens zwei Sperrglieder (30, 32) voneinander umfaßt.

2. Instrument nach Anspruch 1, **dadurch gekennzeichnet, daß** die mindestens eine Entsperrungsvorrichtung (34, 35) plastisch verformbar ist.

3. Instrument nach einem der Ansprüche 1 oder 2, **dadurch gekennzeichnet, daß** die mindestens eine Entsperrungsvorrichtung eine Lösevorrichtung (34, 35) zum irreversiblen Lösen des mindestens einen Sperrglieds (32) vom Instrument (10) umfaßt.

4. Instrument nach Anspruch 3, **dadurch gekennzeichnet, daß** die Lösevorrichtung eine Sollbruchstelle (34, 35) umfaßt.

5. Instrument nach einem der voranstehenden Ansprüche, **dadurch gekennzeichnet, daß** die mindestens eine Entsperrungsvorrichtung eine Sollknickstelle (34, 35) umfaßt.

6. Instrument nach einem der voranstehenden Ansprüche, **dadurch gekennzeichnet, daß** das chirurgische Instrument (10) zwei Betätigungsglieder (12, 14) umfaßt und daß an jedem der beiden Betätigungsglieder (12, 14) ein Sperrglied (30, 32) angeordnet ist.

7. Instrument nach einem der voranstehenden Ansprüche, **dadurch gekennzeichnet, daß** das mindestens eine Betätigungsglied eine mit dem mindestens einen Werkzeugelement (16, 18) starr verbundene Branche (12, 14) ist.

8. Instrument nach Anspruch 6, **dadurch gekennzeichnet, daß** die mindestens eine Entsperrungsvorrichtung (34, 35) eines der beiden Sperrglieder (32) und eines der beiden Betätigungsglieder (14) verbindet.

9. Instrument nach einem der voranstehenden Ansprüche, **dadurch gekennzeichnet, daß** die mindestens eine Entsperrungsvorrichtung (34, 35) aus Kunststoff hergestellt ist.

10. Instrument nach einem der voranstehenden Ansprüche, **dadurch gekennzeichnet, daß** das mindestens eine Sperrglied (30, 32) aus Kunststoff hergestellt ist.

11. Instrument nach einem der voranstehenden Ansprüche, **dadurch gekennzeichnet, daß** die mindestens eine Entsperrungsvorrichtung (34, 35) an eines der mindestens zwei Betätigungselemente (14) und/oder an eines der beiden Sperrglieder (32) angespritzt ist.

12. Instrument nach einem der voranstehenden Ansprüche, **dadurch gekennzeichnet, daß** die mindestens eine Entsperrungsvorrichtung (34, 35) eine Querschnittsfläche aufweist, die kleiner als die halbe Querschnittsfläche des mit ihr verbundenen Sperrglieds (32) ist.

13. Instrument nach einem der voranstehenden Ansprüche, **dadurch gekennzeichnet, daß** die mindestens eine Entsperrungsvorrichtung zwei voneinander beabstandete, eines der beiden Sperrglieder (32) mit dem mindestens einen Betätigungselement (14) verbindende Verbindungsstege (34, 35) umfaßt.

14. Instrument nach einem der voranstehenden Ansprüche, **dadurch gekennzeichnet, daß** jedes Sperrglied (30, 32) mindestens einen in Richtung auf das damit verbundene Betätigungsglied (12, 14) weisenden Rückhaltevorsprung (38) aufweist, und daß der mindestens eine Rückhaltevorsprung (38) des einen Betätigungsglieds (12) und der mindestens eine Rückhaltevorsprung (38) des anderen Betätigungsglieds (14) in einer Sperrstellung der Sperrvorrichtung (28) aneinander anliegen.

15. Instrument nach Anspruch 14, **dadurch gekennzeichnet, daß** mindestens ein Sperrglied (30, 32) mehrere in Richtung auf die damit verbundene Branche (12, 14) weisende Rückhaltevorsprünge (38) aufweist.

16. Instrument nach einem der voranstehenden Ansprüche, **dadurch gekennzeichnet, daß** ein Grundkörper des Instruments (10) aus Metall hergestellt und wahlweise mindestens teilweise von einem Kunststoff bedeckt ist.

17. Instrument nach einem der Ansprüche 1 bis 15, **dadurch gekennzeichnet, daß** das Instrument (10) aus Kunststoff hergestellt ist.

## Claims

1. Surgical instrument (10) with at least two instrument elements (16, 18) which can move relative to one another, with at least one actuating element (12, 14) associated with the at least two instrument elements (16, 18) and with a locking device (28) for fixing a relative position of the at least two instrument elements (16, 18), wherein the locking device (28) comprises two locking members (30, 32) which slide on one another and wherein one locking member (30, 32) is associated with one of the at least two instrument elements (16, 18) and the other locking element (30, 32) with another of the at least two instrument elements (16, 18), **characterised in that** the instrument (10) comprises at least one unlocking device (34, 35) for irreversibly unlocking the at least two locking members (30, 32) from one another.

2. Instrument according to Claim 1, **characterised in that** the at least one unlocking device (34, 35) is plastically deformable.

3. Instrument according to either of Claims 1 and 2, **characterised in that** the at least one unlocking device comprises a release device (34, 35) for irreversibly releasing the at least one locking member (32) from the instrument (10).

4. Instrument according to Claim 3, **characterised in that** the release device comprises a predetermined breaking point (34, 35).

5. Instrument according to any one of the preceding Claims, **characterised in that** the at least one unlocking device comprises a predetermined bending point (34, 35).

6. Instrument according to any one of the preceding Claims, **characterised in that** the surgical instrument (10) comprises two actuating members (12, 14), and that a locking member (30, 32) is disposed on each of the two actuating members (12, 14).

7. Instrument according to any one of the preceding Claims, **characterised in that** the at least one actuating member is a branch (12, 14) which is rigidly connected to the at least one instrument element (16, 18).

8. Instrument according to Claim 6, **characterised in that** the at least one unlocking device (34, 35) connects one of the two locking members (32) and one of the two actuating members (14).

9. Instrument according to any one of the preceding Claims, **characterised in that** the at least one unlocking device (34, 35) is made of plastics material.

10. Instrument according to any one of the preceding Claims, **characterised in that** the at least one locking member (30, 32) is made of plastics material.

11. Instrument according to any one of the preceding Claims, **characterised in that** the at least one unlocking device (34, 35) is injection-moulded onto one of the at least two actuating elements (14) and/or onto one of the two locking members (32).

12. Instrument according to any one of the preceding Claims, **characterised in that** the at least one unlocking device (34, 35) has a cross-sectional area which is smaller than half the cross-sectional area of the locking member (32) which is connected to it.

13. Instrument according to any one of the preceding Claims, **characterised in that** the at least one unlocking device comprises two connecting webs (34, 35) which are spaced apart and connect one of the two locking members (32) to the at least one actuating element (14).

14. Instrument according to any one of the preceding Claims, **characterised in that** each locking member (30, 32) has at least one retaining projection (38) which points in the direction of the actuating member (12, 14) which is connected thereto, and that the at least one retaining projection (38) of one actuating member (12) and the at least one retaining projection (38) of the other actuating member (14) lie against one another when the locking device (28) is in a locking position.

15. Instrument according to Claim 14, **characterised in that** at least one locking member (30, 32) has a plurality of retaining projections (38) which point in the direction of the branch (12, 14) which is connected thereto.

16. Instrument according to any one of the preceding Claims, **characterised in that** a base body of the instrument (10) is made of metal and optionally covered at least partly by a plastics material.

17. Instrument according to any one of Claims 1 to 15, **characterised in that** the instrument (10) is made of plastics material.

## Revendications

1. Instrument chirurgical (10) avec au moins deux éléments d'outil (16, 18) déplaçables l'un par rapport à l'autre, avec au moins un élément de manoeuvre (12, 14) associé aux au moins deux éléments d'outil (16, 18) et avec un dispositif de verrouillage (28) pour la fixation d'une position relative des au moins deux éléments d'outil (16, 18), dans lequel le dispositif de verrouillage (28) comporte deux éléments de verrouillage (30, 32) glissant l'un à côté de l'autre et dans lequel un élément de verrouillage (30, 32) est associé à l'un des au moins deux éléments d'outil (16, 18) et l'autre élément de verrouillage (30, 32) est associé à l'autre des au moins deux éléments d'outil (16, 18), **caractérisé en ce que** l'instrument (10) comporte au moins un dispositif de déverrouillage (34, 35) pour le déverrouillage irréversible des au moins deux éléments de verrouillage (30, 32).

2. Instrument selon la revendication 1, **caractérisé en ce que** le au moins un dispositif de déverrouillage (34, 35) est déformable plastiquement.

3. Instrument selon l'une quelconque des revendications 1 ou 2, **caractérisé en ce que** le au moins un dispositif de déverrouillage comporte un dispositif de desserrage (34, 35) pour le desserrage irréversible du au moins un élément de verrouillage (32) de l'instrument (10).

4. Instrument selon la revendication 3, **caractérisé en ce que** le dispositif de desserrage comporte un point destiné à la rupture (34, 35).

5. Instrument selon l'une quelconque des revendications précédentes, **caractérisé en ce que** le au moins un dispositif de déverrouillage comporte un point destiné au pliage (34, 35).

6. Instrument selon l'une quelconque des revendications précédentes, **caractérisé en ce que** l'instrument chirurgical (10) comprend deux éléments de manoeuvre (12, 14) et **en ce qu'**un élément de verrouillage (30, 32) est disposé sur chacun des deux éléments de manoeuvre (12, 14).

7. Instrument selon l'une quelconque des revendications précédentes, **caractérisé en ce que** le au moins un élément de manoeuvre est une branche (12, 14) reliée rigidement avec le au moins un élément d'outil (16, 18).

8. Instrument selon la revendication 6, **caractérisé en ce que** le au moins un dispositif de déverrouillage (34, 35) relie l'un des deux éléments de verrouillage (32) et l'un des deux éléments de manoeuvre (14).

9. Instrument selon l'une quelconque des revendications précédentes, **caractérisé en ce que** le au moins un dispositif de déverrouillage (34, 35) est fait en matière plastique.

10. Instrument selon l'une quelconque des revendications précédentes, **caractérisé en ce que** le au moins un élément de verrouillage (30, 32) est fait en matière plastique.

11. Instrument selon l'une quelconque des revendications précédentes, **caractérisé en ce que** le au moins un dispositif de déverrouillage (34, 35) est injecté sur l'un des au moins deux éléments de manoeuvre (14) et/ou sur l'un des deux éléments de verrouillage (32).

12. Instrument selon l'une quelconque des revendications précédentes, **caractérisé en ce que** le au moins un dispositif de déverrouillage (34, 35) présente une aire de section transversale qui est plus petite que la moitié de l'aire de section transversale de l'élément de verrouillage (32) auquel elle est reliée.

13. Instrument selon l'une quelconque des revendications précédentes, **caractérisé en ce que** le au moins un dispositif de déverrouillage comporte deux nervures de liaison (34, 35) espacées l'une de l'autre qui relient l'un des deux éléments de verrouillage (32) avec le au moins un élément de manoeuvre (14).

14. Instrument selon l'une quelconque des revendications précédentes, **caractérisé en ce que** chaque élément de verrouillage (30, 32) comporte au moins un épaulement de retenue (38) orienté en direction de l'élément de manoeuvre (12, 14) auquel il est relié et **en ce que** le au moins un épaulement de retenue (38) d'un élément de manoeuvre (12) et le au moins un épaulement de retenue (38) de l'autre élément de manoeuvre (14) sont adjacents dans une position de verrouillage du dispositif de verrouillage (28).

15. Instrument selon la revendication 14, **caractérisé en ce qu'**au moins un élément de verrouillage (30, 32) comporte plusieurs épaulements de retenue (38) orientés en direction de la branche (12, 14) à laquelle ils sont reliés.

16. Instrument selon l'une quelconque des revendications précédentes, **caractérisé en ce qu'**un corps de base de l'instrument (10) est fait en métal et est recouvert au choix au moins partiellement par une matière plastique.

17. Instrument selon l'une quelconque des revendications 1 à 15, **caractérisé en ce que** l'instrument (10) est fait en matière plastique.
